# FASCICULE DE BREVET EUROPEEN

(11) **EP 3 151 783 B1**
(45) Date de publication et mention de la délivrance du brevet: **27.03.2019**
(21) Numéro de dépôt: 15733803.9
(22) Date de dépôt: 04.06.2015
(51) Int. Cl.: A61F 2/01, A61F 2/24

(54) **DISPOSITIF D'INTRODUCTION TRANSCATHETER DANS LA RACINE AORTIQUE AU NIVEAU DE LA JONCTION SINO TUBULAIRE**
VORRICHTUNG FÜR DEN KATHETEREINSATZ IN DIE AORTENWURZEL AN DER SINOTUBULAREN VERBINDUNG
DEVICE FOR TRANSCATHETER INSERTION INTO THE AORTIC ROOT AT THE SINOTUBULAR JUNCTION

(30) Priorité: 05.06.2014 FR 1455144
(43) Date de publication de la demande: 12.04.2017
(73) Titulaire: AORTICLAB SARL, 1073 Savigny (CH); Centre Hospitalier Universitaire De Saint Etienne, 42055 Saint-Etienne Cedex 2 (FR)
(72) Inventeur: PAIN, Bernard, 43120 Monistrol-sur-Loire (FR); VOLA, Marco, 42270 Saint-Priest-en-Jarez (FR); PASQUINO, Enrico, 1073 Savigny (CH)
(74) Mandataire: Grosfillier, Philippe
(86) Numéro de dépôt international: PCT/FR2015/051488
(87) Numéro de publication internationale: WO 2015/185870

(56) Documents cités:
- WO-A2-02/47539
- WO-A2-2004/043293

## Description

L'invention concerne le domaine technique de la cardiologie interventionnelle et de la chirurgie endovasculaire et mini invasive et concerne plus particulièrement un dispositif introducteur pour intervention par voie transcathéter.

L'invention trouve une application avantageuse, qui ne doit toutefois pas être considérée comme limitative pour tout type d'intervention chirurgicale, par exemple pour un remplacement d'une valve ou d'une implantation de la valve par voie transcathéter, plus généralement connu pour un homme du métier sous le nom de TAVI.

Toute intervention par voie transcathéter nécessite pour l'opérateur, de prendre de très grandes précautions, étant donné que pour une intervention cardiaque par exemple, la circulation du sang n'est pas déviée, ce qui n'est pas le cas, par exemple, pour une opération à coeur ouvert où il y a une circulation extracorporelle du sang.

La génération de débris pendant la procédure de TAVI à partir de la valve aortique peut générer une embolisation coronarienne post-opératoire, qui conduit à un infarctus péri procédure, tandis que l'embolisation cérébrale peut causer des AVC iatrogènes.

On sait, de plus, que l'implantation d'une valve aortique par TAVI à travers une valve aortique native très calcifiée génère souvent des fuites péri valvulaires susceptibles de mettre en danger la vie du patient à moyen terme. Ce dernier phénomène est dû à l'irrégularité des calcifications qui produisent une adhésion imparfaite de la bioprothèse du TAVI avec l'anneau aortique du patient.

La demande de brevet WO 2004/043293 décrit un dispositif de traitement d'une valve aortique, sans circulation extra-corporelle. Le dispositif est introduit à travers une canule

Afin d'obtenir de meilleurs résultats par TAVI, il est apparu important de poser la valve sur une surface la plus régulière possible, afin d'éviter une distorsion susceptible de rendre l'ouverture de la prothèse incomplète et, également, d'améliorer l'affrontement entre anneau aortique natif et bioptothèse. Ceci avec l'objectif de minimiser les fuites péri valvulaires qui affectent le TAVI et qui impactent sur la survie des patients à moyen terme.

A partir de cet état de la technique, pour obtenir, par exemple, un remodelage du site d'implantation pouvant aller de la décalcification transcathéter moyenne jusqu'à la suppression totale des volets valvulaires natifs et avec, pour objectif, de permettre en toute sécurité différentes procédures et interventions par voie transcathéter, il est apparu important de pouvoir mettre en place temporairement un dispositif apte à remplacer la fonction de la valve native. En effet un des problèmes majeurs de la réalisation de la décalcification de la valve aortique transcathéter à coeur battant et sans circulation extra-corporelle est celui d'éviter la migration des débris de calcium dans la racine aortique et en aval, dans la crosse aortique. Ce phénomène est sans doute plus important avec une procédure de décalcification transcathéter que pendant une procédure de TAVI standard, qui se pratique actuellement sans la décalcification de la valve aortique.

Pour résoudre ce problème et atteindre ces objectifs, il a été conçu et mis au point un dispositif introduit par voie transcathéter dans la racine aortique au niveau de la jonction sino tubulaire, au moyen d'un guide de fil et d'un cathéter, avec un moyen de protection des tissus environnant lors de la mise en place dudit cathéter. L'invention et ses différents modes de réalisation sont délimités par les revendications ci-jointes.

Selon l'invention, le dispositif comprend un ensemble faisant office de filtre embolique valvulé, monté avec capacité de coulissement guidé à l'intérieur dudit cathéter, ledit ensemble présentant des agencements aptes à réaliser, dans la racine aortique, une enceinte de sécurité assurant une fonction de valve et une fonction de protection contre les accidents d'embolie.

Il résulte de ces caractéristiques que le dispositif rende possible de réaliser différentes interventions par voie transcathéter, par exemple, enlever les tissus calcifiés et les végétations dans et dessus les volets de la valve aortique, mettre en place tout type de valve, ... en observant que l'intervention s'effectue en condition de circulation physiologique et non pas sous circulation extracorporelle.

Pour résoudre le problème posé de créer cette enceinte de sécurité, les agencements de l'ensemble faisant office de filtre embolique valvulé, comprennent un corps tubulaire assujetti à l'une de ses extrémités à une partie apte à être à volonté, soit déployée à l'extérieur du cathéter, soit rétractée à l'intérieur du cathéter, ladite partie présentant des agencements de filtration combinés avec des moyens aptes à reproduire une fonction de valve temporaire correspondant à une ouverture pendant la phase systolique et a une fermeture pendant la phase diastolique afin d'empêcher toute régurgitation sanguine dans une position déployée de ladite partie pour couvrir la valve aortique native avec une assise sécurisée dans la racine aortique au niveau des sinus de Valsalva sans obstruer le flux sanguin.

Pour résoudre le problème posé de reproduire les commissures de la valve native, la partie du filtre embolique est de forme générale conique et présente des coques décalées angulairement dont les contours enveloppent les commissures de la valve native, lesdites coques étant montées en combinaison avec une membrane filtrante.

Il résulte de ces dispositions que le cône peut se placer sur le plancher de la racine aortique, au contact du bord de l'anneau aortique, se déplacer en restant au-dessous des ostium coronaires et en même temps contourner les commissures de la valve native.

Le positionnement du filtre embolique valvulé sur la racine aortique aux environs des commissures est très important pour éviter les insuffisances aortiques aigues pendant l'intervention transcathéter de décalcification.

Dans une forme de réalisation de la partie active du filtre embolique valvulé, la membrane filtrante comprend, d'une part, une couche inférieure constituée d'un réseau maillé avec une porosité adaptée pour bloquer des débris de tissu, tout en permettant le passage du flux sanguin et, d'autre part, une couche supérieure réalisée dans un matériau polymère souple et extensible pour agir comme une valve temporaire, par simple déformation déterminée par la pression systolique.

Les couches sont fixées ensemble par soudure à la base de la forme conique et sur son pourtour, ladite soudure étant souple et spongieuse pour fournir une assise optimale du filtre valvulaire sur la base de la racine aortique.

La couche inférieure maillée est fixée à l'extrémité du corps et en prolongement de ce dernier, tandis que la couche supérieure faisant office de valve, est libre et reste ouverte sur le dessus de la forme conique.

Selon une autre caractéristique, le cathéter présente une extrémité radio opaque apte à protéger les tissus environnant lors de l'introduction et de la navigation dans l'aorte. Cette extrémité est rétractable à l'intérieur du corps tubulaire de l'ensemble faisant office de filtre embolique. Avantageusement l'extrémité est un ballonnet gonflable rempli d'une solution radio opaque stérile.

L'invention est exposée ci-après plus en détail à l'aide des figures des dessins annexés dans lesquels :
- La figure 1 est une vue partielle en perspective montrant notamment l'ensemble faisant office de filtre embolique valvulé.
- La figure 2 est une vue correspondant à la figure 1 avec une coupe partielle.
- Les figures 3 à 20 montrent les principales étapes pour une intervention transcathéter au moyen du dispositif selon l'invention dans le cas d'une application à une valve aortique, une telle application ne devant pas être considérée comme rigoureusement limitative.

Le dispositif selon l'invention comprend un ensemble (E) faisant office de filtre embolique valvulé, monté avec capacité de coulissement guidé à l'intérieur d'un cathéter (1). Comme il sera indiqué dans la suite de la description, le cathéter (1) est équipé de l'ensemble (E) introduit dans la racine aortique (RA) au moyen d'un guide fil (g) comme cela est couramment pratiqué pour ce type d'intervention.

L'ensemble filtrant (E) présente des agencements aptes à réaliser dans la racine aortique (RA), une enceinte de sécurité assurant une fonction de valve et une fonction de protection contre les accidents d'embolie. Cet ensemble (E) comprend un corps (2) monté à libre coulissement à l'intérieur du cathéter (1). Bien évidemment, le cathéter (1) et le corps (2) sont réalisés dans un matériau souple.

Le corps tubulaire (2) est assujetti à l'une de ses extrémités à une partie (2a) de forme générale conique et apte à constituer l'enceinte de sécurité en tant que telle. Cette partie (2a) peut, à volonté, soit être déployée à l'extérieur du cathéter (1) soit, rétractée à l'intérieur dudit cathéter (1). Cette partie (2a) présente des agencements de filtration combinés avec des moyens aptes à reproduire la fonction de valve temporaire correspondant à une ouverture pendant la phase systolique et à une fermeture pendant la phase diastolique.

Le but recherché est donc d'empêcher toute régurgitation sanguine dans une position déployée de cette partie (2a) qui constitue l'enceinte de sécurité avec, pour objectif de couvrir la valve aortique native avec une assise sécurisée dans la racine aortique et, plus précisément, à la jonction entre l'anneau et l'origine des sonus de Valsalva sans obstruer le flux sanguin.

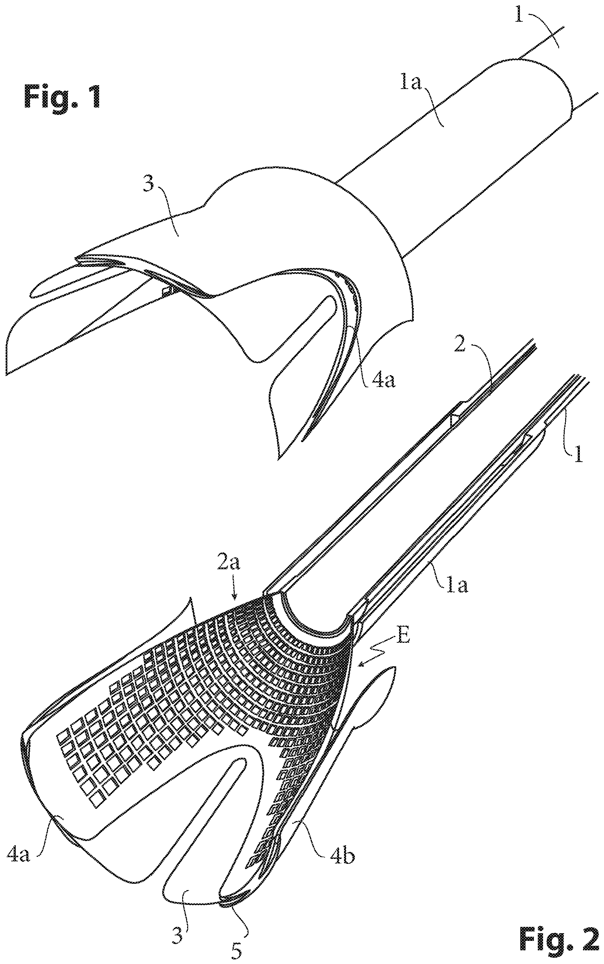

Comme le montrent les figures des dessins, notamment les figures 1 et 2, la partie (2a) du filtre embolique est de forme générale conique et présente des coques (3) décalées angulairement. Par exemple, ces coques sont décalées de 120° afin de reproduire la position des commissures de la valve native. La base de la partie conique constituée par les coques (3) peut, par conséquent, se placer sur le plancher de la racine aortique, se déplacer sous l'ostium coronaire et, en même temps, contourner les trois commissures de la valve native. A noter que dans le cas d'une valve aortique bicuspide, la forme générale conique peut être adaptée et présenter seulement deux coques.

Ces caractéristiques permettent un bon positionnement du filtre embolique valvulé, ce qui est important.

Les coques (3) sont montées en combinaison avec une membrane filtrante (4). Cette membrane filtrante (4) comprend une couche (4a) dite inférieure, constituée d'un réseau maillé avec porosité adaptée pour bloquer les éventuels débris de tissu tout en permettant le passage du flux sanguin. Cette empreinte filtrante (4) présente, par ailleurs, une autre couche (4b) dite supérieure, réalisée dans un matériau polymère fin, souple et extensible afin d'agir comme une valve temporaire par simple déformation.

Les couches (4a), (4b) sont fixées ensemble à la base de la partie conique constituée par les coques (3). Cette fixation est réalisée par soudure (5) sur le pourtour de la base de la partie conique (2a). La soudure est souple et spongieuse pour fournir une assise optimale du filtre valvulaire à la base de la racine aortique.

La couche inférieure maillée (4a) est fixée à l'extrémité du corps (2) et en prolongement de ce dernier par tout moyen connu et approprié. Par exemple, cette fixation peut également être réalisée par soudure. La couche supérieure (4b) faisant office de valve est, par conséquent, libre et reste ouverte sur le dessus de la forme conique.

Le cathéter (1) présente une extrémité radio opaque apte à protéger les tissus environnants lors de son introduction et de sa navigation dans l'aorte. Comme il sera indiqué dans la suite de la description, l'extrémité est rétractable à l'intérieur de l'ensemble faisant office de filtre embolique et, par conséquent, rétractable à l'intérieur du corps (2) de ce dernier et du cathéter (1). Avantageusement, cette extrémité est constituée par un ballonnet (6) gonflable rempli d'une solution radio opaque stérile.

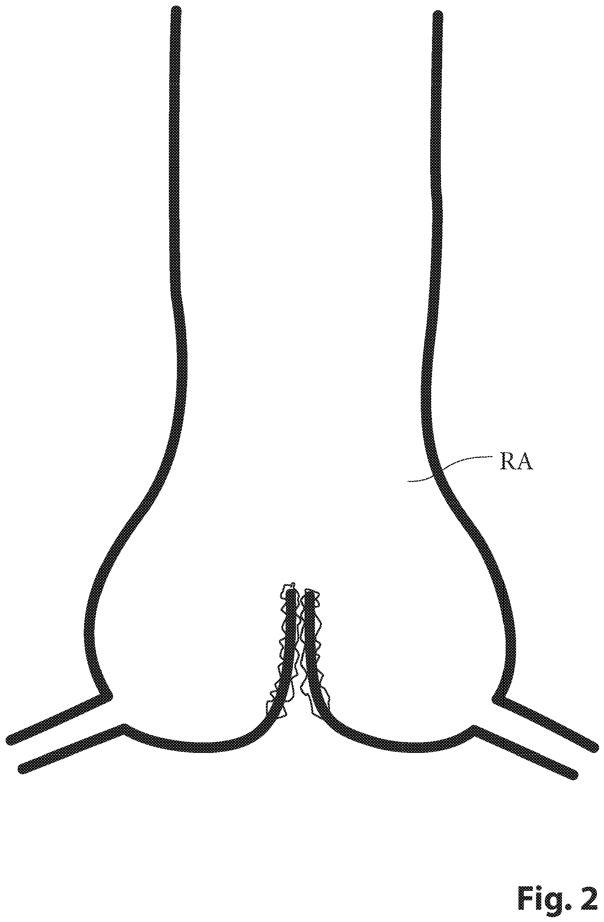

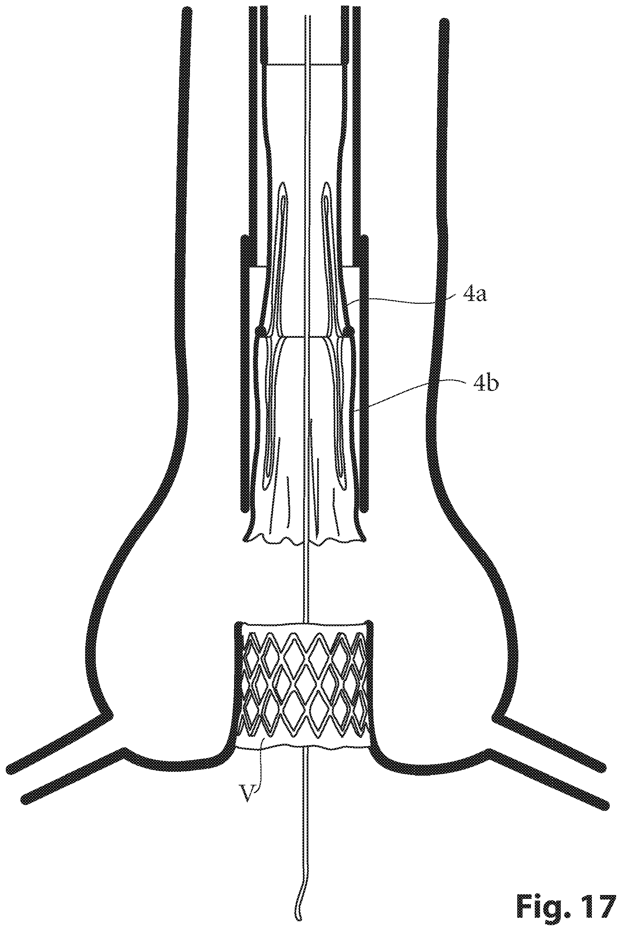

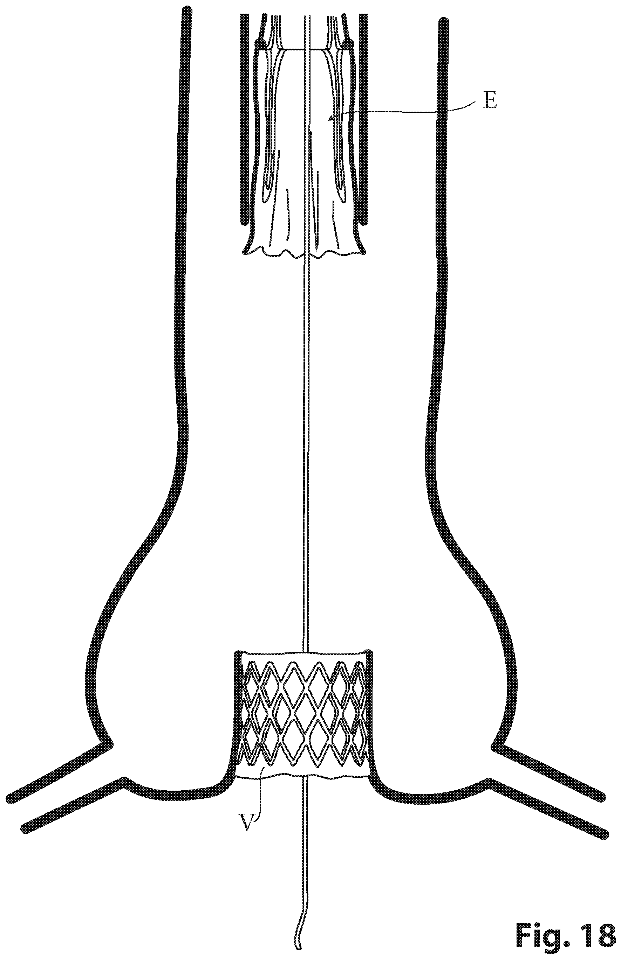

Il convient de se référer aux figures 3 à 20 montrant les différentes séquences pour la mise en place d'une valve aortique, en observant que le dispositif peut être appliqué pour d'autres valves cardiaques telles que tricuspides, mitrales, pulmonaires mais également pour l'introduction d'instruments tel qu'un décalcificateur afin d'obtenir un remodelage du site d'implantation.

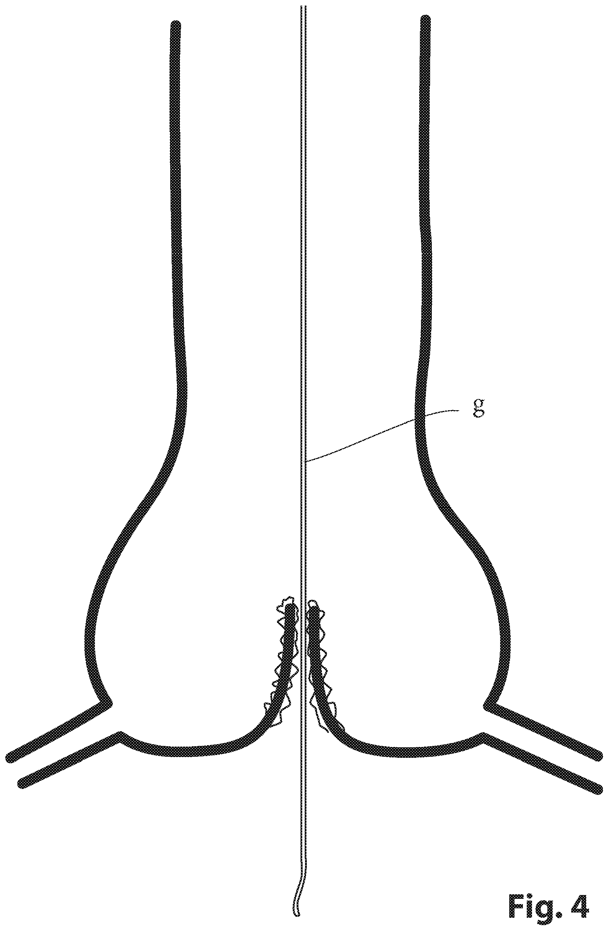

La figure 3 montre la racine aortique tandis que la figure 4 montre la même racine après mise en place du guide fil (g) qui, de manière connue, est engagé afin de traverser la valve aortique sténosée.

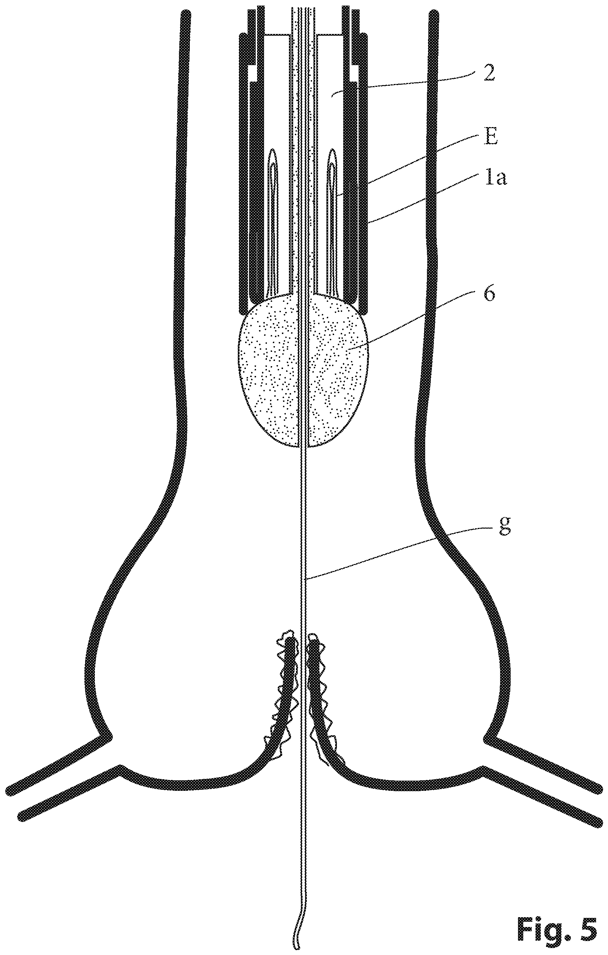

L'ensemble du dispositif introducteur incluant le cathéter (1) équipé de l'ensemble (E) faisant office de filtre embolique valvulé et du ballonnet (6) est introduit dans l'aorte descendante au moyen du guide fil (g), figure 5. A noter que l'ensemble du dispositif peut être introduit directement dans la racine aortique avec une jonction directe de l'aorte ascendante suite à un accès transaortique à travers une petite thoracotomie ou au moyen d'un trocart d'endoscopie ou bien par voie transcathéter à travers l'artère fémorale ou à travers un accès d'auto-faisceaux périphériques telles que les artères sous-clavières ou artères auxiliaires.

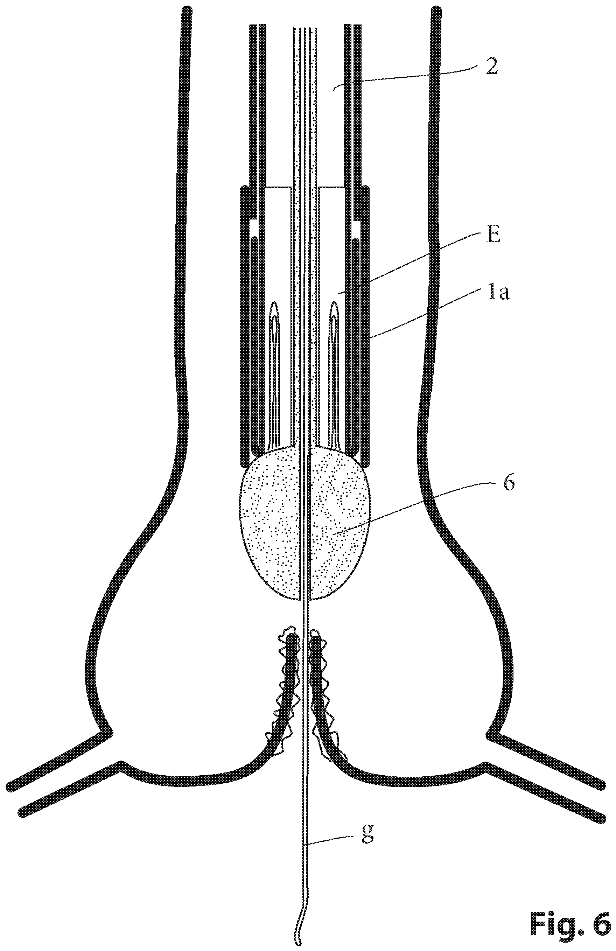

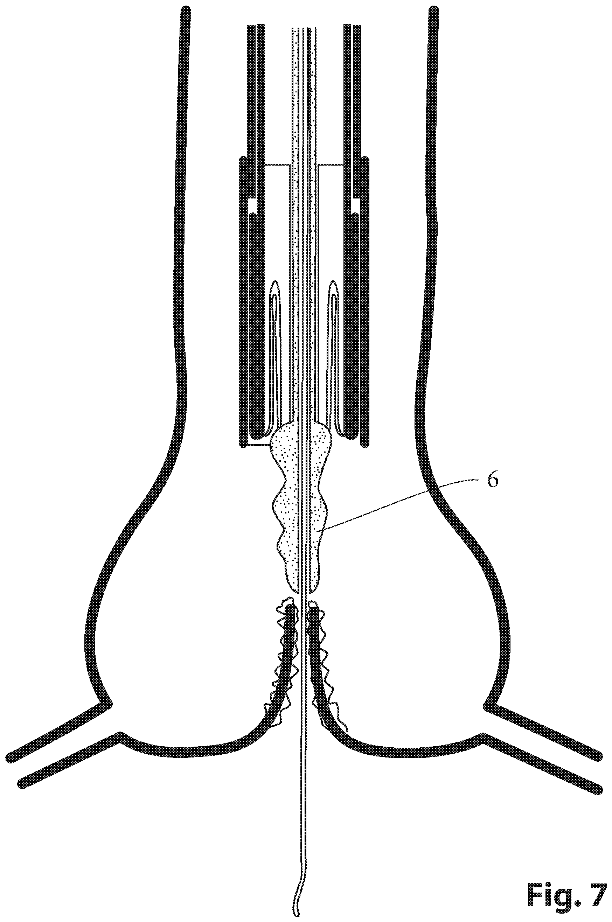

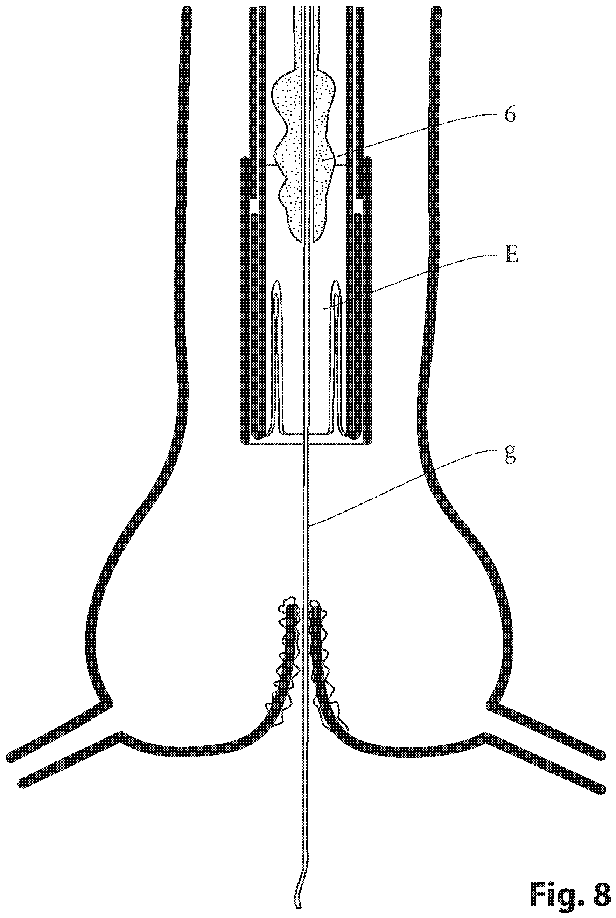

L'ensemble du cathéter introducteur est positionné au niveau de la jonction sino tubulaire, figure 6. Lorsque le dispositif est en position, l'extrémité du ballonnet est dégonflée, figure 7, de manière à ce que son diamètre soit inférieur au diamètre interne du dispositif introducteur notamment du corps tubulaire (2), figure 7. Le ballonnet (6) est alors complètement retiré du dispositif, figure 8.

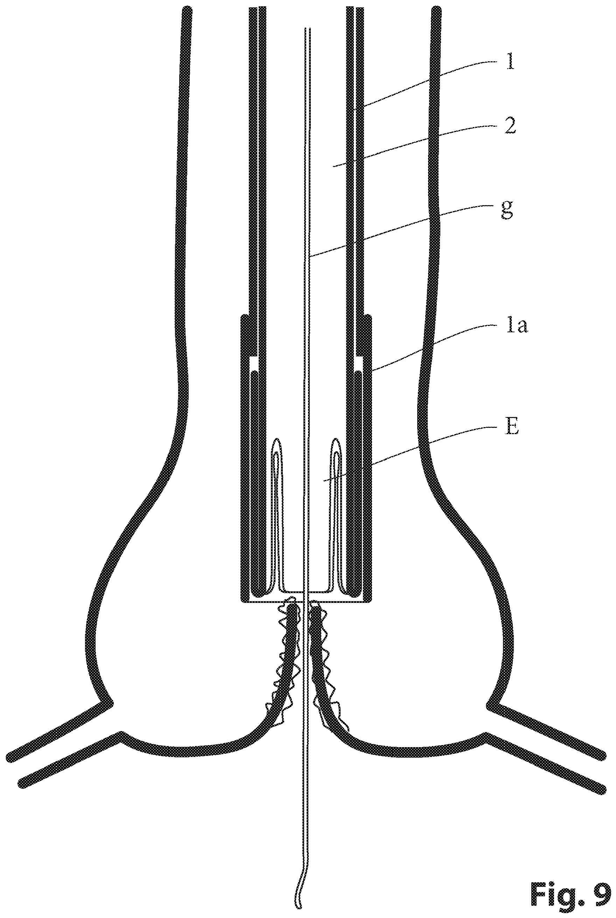

Le dispositif est ensuite poussé au niveau de la racine aortique, figure 9.

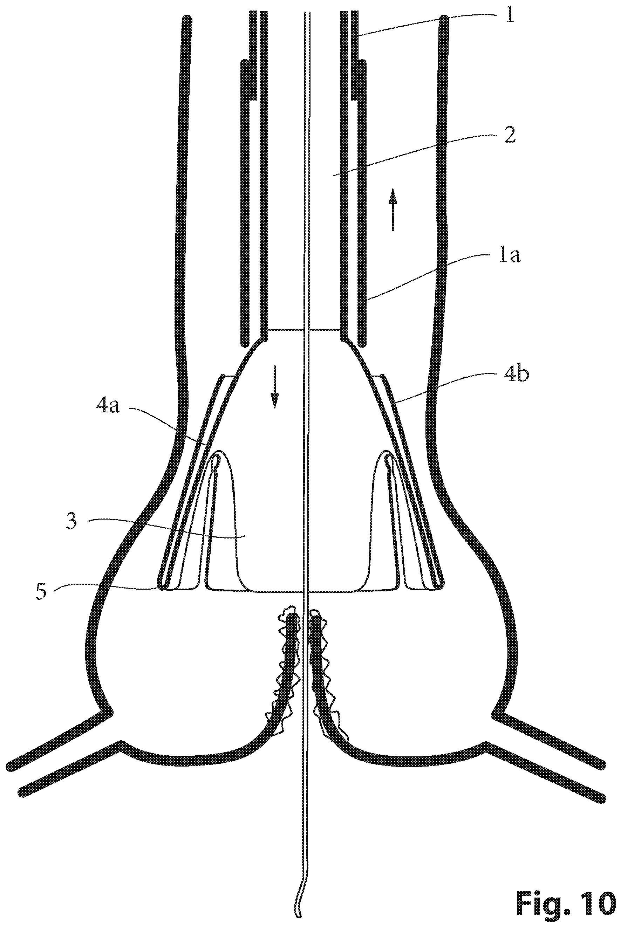

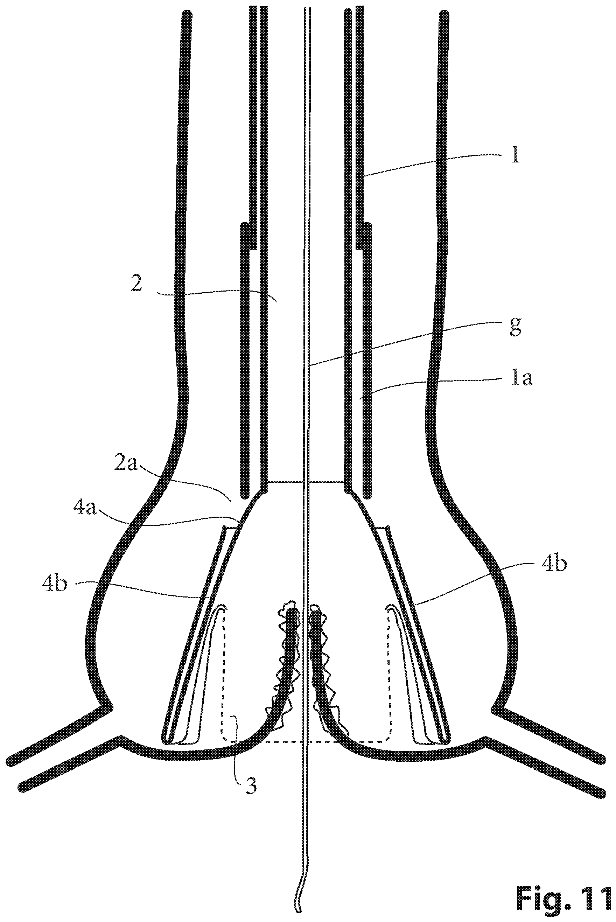

Le cathéter (1) est ensuite retiré (flèche F), figure 10, de manière à ce que la partie de l'extrémité constituant le fourreau (1a) recouvrant l'ensemble (E) du filtre valvulé libère ce dernier qui est partiellement déployé, figure 10. Le corps tubulaire (2) est alors poussé de la racine aortique jusqu'à ce que l'ensemble du filtre, par sa partie externe résultant de la soudure souple, vienne prendre appui dans les sinus Valsalva à proximité de l'ostium coronaire sans obstruer le flux coronarien, figure 11.

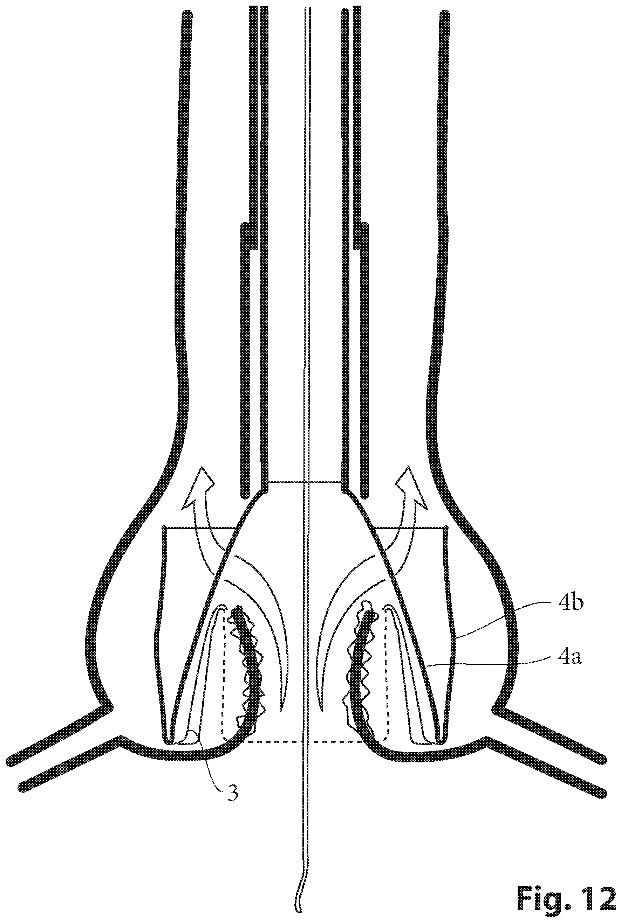

La couche supérieure (4b) de la membrane réalisée dans un matériau polymère souple et extensible, agit comme une valve garantissant une complète fonction de valve, figure 12, tandis que le réseau maillé de la couche inférieure (4a) bloque les débris de tissu. Les coques (3) s'adaptent, comme indiqué, aux commissures de la valve native permettant une bonne étanchéité et une assise totale du dispositif sur le plancher de la racine aortique.

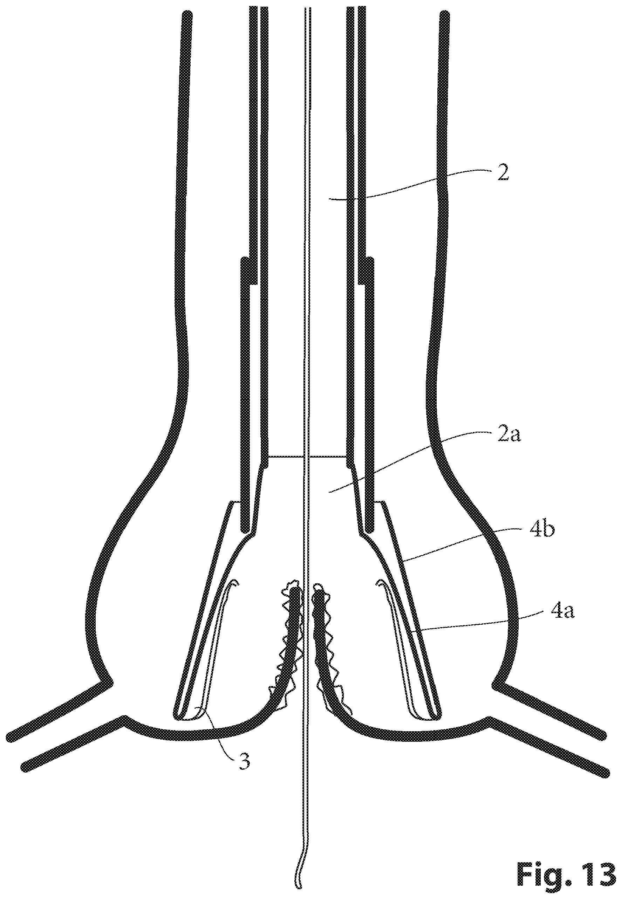

Comme le montre la figure 13, l'ouverture du filtre conique valvulé peut être modifiée en agissant sur le cathéter (1) dont l'extrémité (1a) forme un fourreau coopérant avec ledit filtre pour modifier l'ouverture du filtre de manière restrictive, figure 13.

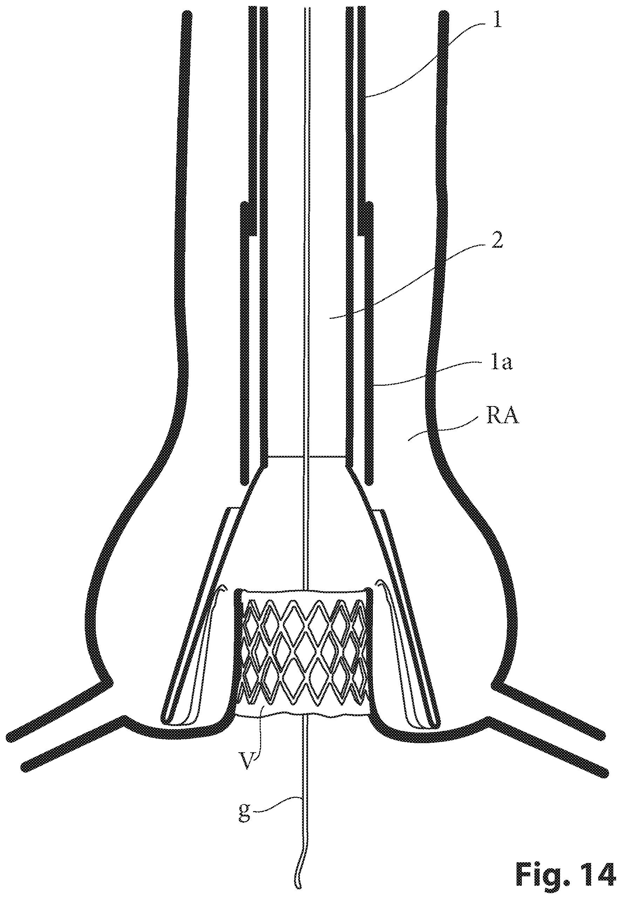

Après avoir vérifié la position stable du cathéter (1), il est possible d'introduire, par voie cathéter, le dispositif médical choisi, par exemple, une valve (V), figure 14. Si nécessaire, préalablement à la mise en place de la valve, il est possible de réaliser une décalcification par tout moyen connu et approprié.

A noter que si le tissu des feuilles à enlever a été limité et que la régurgitation est moyenne à modérée, le dispositif introducteur peut être enlevé et la valve transcathéter peut être implantée.

A contrario, si l'ablation des tissus a été complète avec retrait total des volets, il est important de maintenir en place le dispositif introducteur pour garantir la fonction de valve temporaire à défaut d'une insuffisance aortique qui pourrait constituer une menace à la vie du patient au cours de l'intervention chirurgicale.

Quand la fonction valvule du filtre conique est terminée, le dispositif peut alors être retiré.

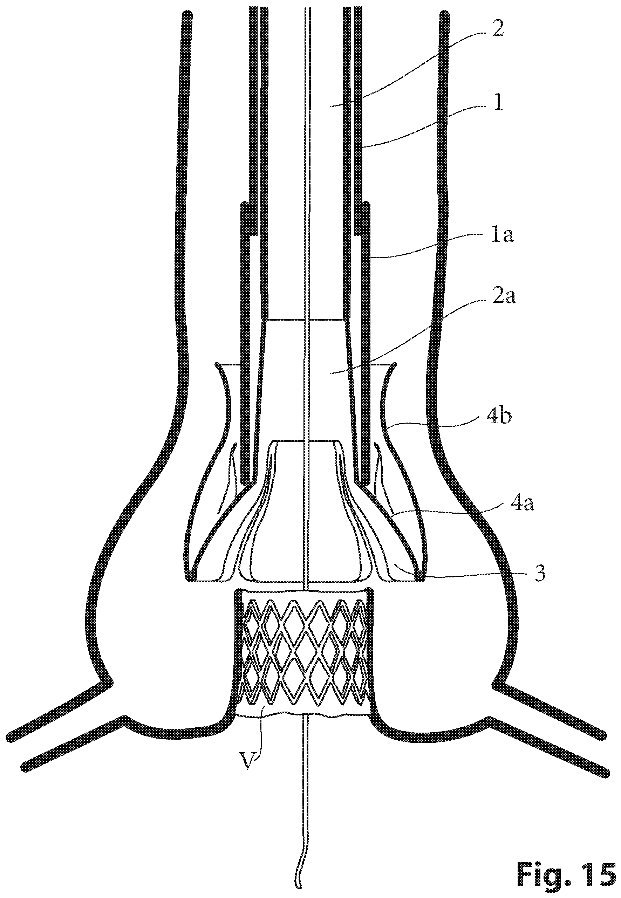

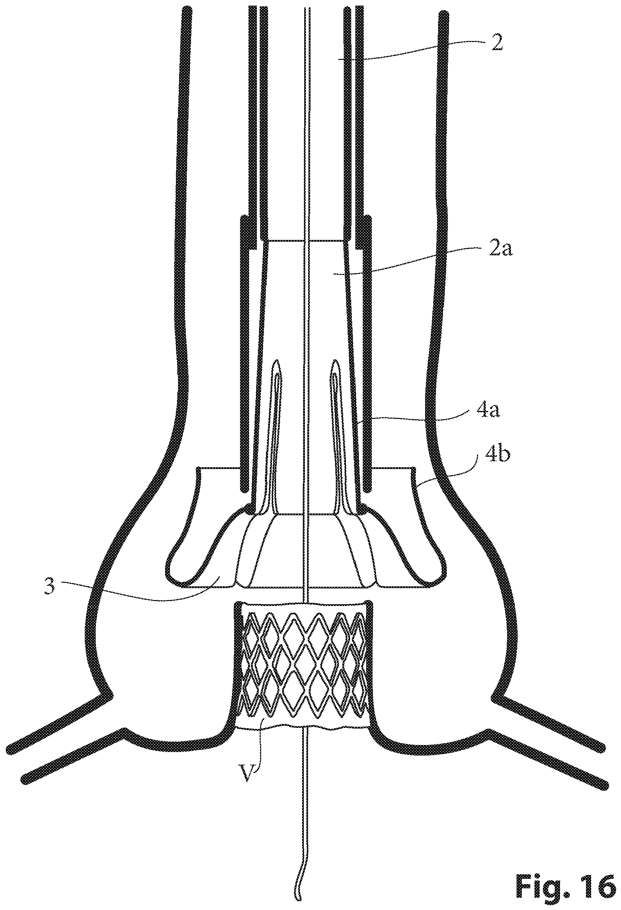

Le retrait du dispositif consiste en la fermeture de la membrane du filtre en s'assurant que les débris prisonniers ne sont pas embolisés. Le filtre valvulé est doucement retiré de la racine de la valve aortique, figure 15, et est progressivement réintroduit à l'intérieur du cathéter. Le fourreau (1a) du cathéter (1) est glissé vers le bas entre le matériau polymère de la membrane et le filtre polymère, figures 16, 17 et 18, jusqu'à atteindre l'extrémité de la partie conique en la fermant progressivement jusqu'à renverser les coques (3) qui sont rétractées à l'intérieur du cathéter (1) par-dessus le matériau polymère en le faisant glisser afin de purger les débris capturés dans le filtre.

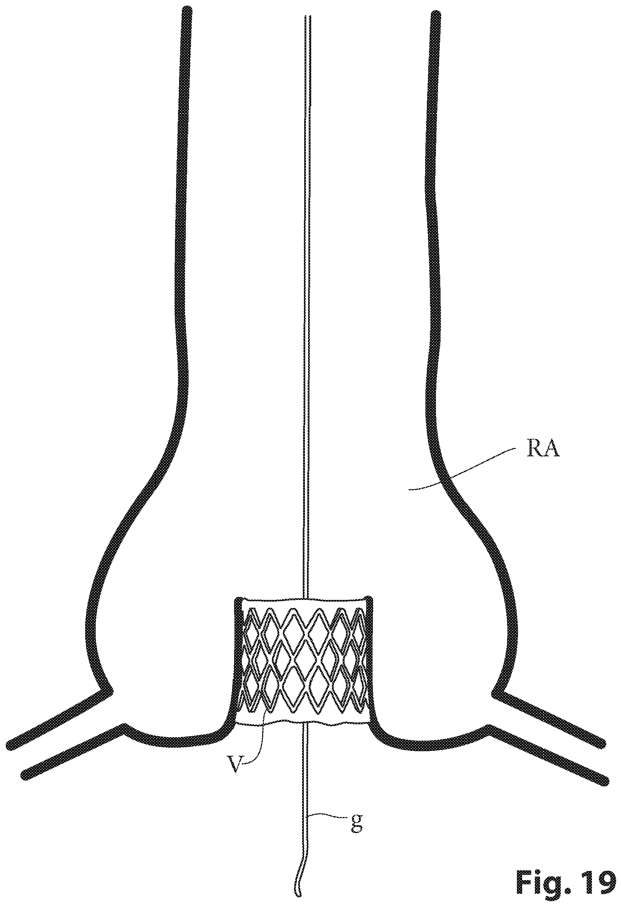

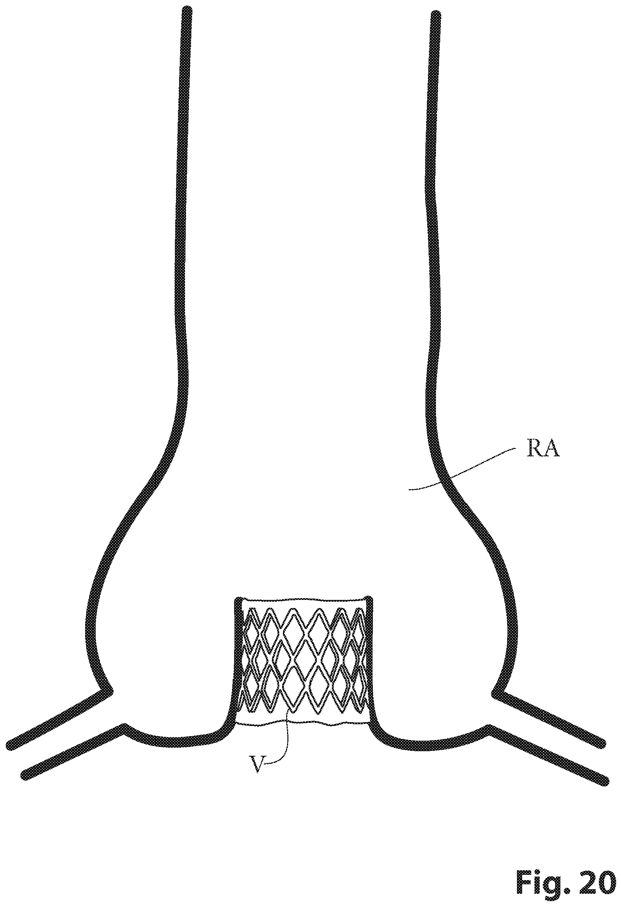

Lorsque la procédure de retrait est terminée et le filtre valvulé complètement fermé, le dispositif peut être retiré en toute sécurité de l'aorte, figure 19. Il suffit ensuite d'enlever le guide fil, figure 20.

Les avantages ressortent bien de la description.

## Revendications

1. Dispositif d'introduction transcathéter dans la racine aortique au niveau de la jonction sino tabulaire au moyen d'un guide de fil et d'un cathéter (1) avec un moyen de protection des tissus environnant, ledit dispositif comprend un ensemble (E) faisant office de filtre embolique valvulé, monté avec capacité de coulissement guidé à l'intérieur dudit cathéter (1), ledit ensemble (E) présentant des agencements aptes à réaliser, dans la racine aortique (AR), une enceinte de sécurité assurant une fonction de valve et une fonction de protection contre les accidents d'embolie, lesdits agencements comprennent un corps tubulaire (2) assujetti, à l'une de ses extrémités, à une partie (2a) apte à être, à volonté, soit déployée à l'extérieur du cathéter (1), soit rétractée à l'intérieur du cathéter (1), ladite partie (2a) présentant des agencements de filtration combinés avec des moyens aptes à reproduire la fonction de valve temporaire correspondant à une ouverture pendant la phase systolique et a une fermeture pendant la phase diastolique afin d'empêcher toute régurgitation sanguine dans une position déployée de ladite partie pour couvrir la valve aortique native avec une assise sécurisée dans la racine aortique au niveau des sinus Valsalva sans obstruer le flux sanguin ; dispositif dans lequel ladite partie (2a) est de forme générale conique et présente des coques (3) décalées angulairement dont les contours enveloppent les commissures de la valve native, lesdites coques étant montées en combinaison avec une membrane filtrante (4), cette dernière comprenant d'une part une couche inférieure (4a) constituée d'un réseau maillé avec une porosité adaptée pour bloquer des débris de tissu tout en permettant le passage du flux sanguin et, d'autre part, une couche supérieure (4b) réalisée dans un matériau polymère souple et extensible pour agir comme une valve temporaire, par simple déformation ; dispositif **caractérisé en ce que** lesdites couches (4a,4b) sont fixées ensemble par soudure à la base de la forme conique et sur son pourtour, ladite soudure étant souple et spongieuse pour fournir une assise optimale du filtre valvulaire sur la base de la racine aortique et pour permettre à l'extrémité de ladite forme conique d'être apte à être fermée progressivement jusqu'à rétractation à l'intérieur du cathéter par-dessus le matériau polymère en le faisant glisser afin de purger les débris capturés lorsque le cathéter (1) est glissé vers le bas entre le matériau polymère de la membrane (4) et le filtre polymère jusqu'à atteindre ladite extrémité de ladite forme conique.

2. Dispositif selon la revendication 1, **caractérisé en ce que** la couche inférieure maillée est fixée à l'extrémité du corps et en prolongement de ce dernier, tandis que la couche supérieure faisant office de valve, est libre et reste ouverte sur le dessus de la forme conique.

3. Dispositif selon la revendication 1 ou 2, **caractérisé en ce que** le cathéter (1) présente une extrémité radio opaque apte à protéger les tissus environnant lors de l'introduction et de la navigation de l'aorte.

4. Dispositif selon la revendication 3, **caractérisé en ce que** l'extrémité est rétractable à l'intérieur du corps tubulaire de l'ensemble (E) faisant office de filtre embolique.

5. Dispositif selon la revendication 4, **caractérisé en ce que** l'extrémité est un ballonnet (6) gonflable rempli de solution radio opaque stérile.

## Patentansprüche

1. Vorrichtung zur Einführung eines Transkatheters in die Aortenwurzel im Bereich der sinotubularen Verbindung mit Hilfe eines Führungsdrahtes und eines Katheters (1) mit einem Mittel zum Schutz des umliegenden Gewebes, wobei die Vorrichtung eine Anordnung (E) aufweist, welche als Herzklappen-Emboliefilter dient, welche mit dem Vermögen zum geführten Gleiten innerhalb des Katheters (1) montiert ist,
wobei die Anordnung (E) Einrichtungen aufweist, welche geeignet sind, in der Aortenwurzel (AR) ein Sicherheitsgehäuse zu erzielen, welches eine Klappenfunktion und eine Schutzfunktion gegen Embolie-Zwischenfälle gewährleistet, wobei die Einrichtungen einen rohrförmigen Körper (2) aufweisen, welcher an einem seiner Enden an einem Abschnitt (2a) befestigt ist, welcher geeignet ist, nach Belieben entweder außerhalb des Katheters (1) entfaltet oder in das Innere des Katheters (1) zurückgezogen zu werden,
wobei der Abschnitt (2a) Filtrierungsanordnungen aufweist, welche mit Mitteln kombiniert sind, welche geeignet sind, die temporäre Ventilfunktion zu reproduzieren, welche einem Öffnen während der systolischen Phase und einen Schließen während der diastolischen Phase entspricht, um jegliche Blut-Regurgitation in einer entfalteten Position des Abschnitts zu verhindern, um die native Aortenklappe mit einem festen Sitz in der Aortenwurzel im Bereich des Valsalva Sinus zu bedecken, ohne den Blutstrom zu blockieren;
wobei bei der Vorrichtung der Abschnitt (2a) von allgemeiner konischer Form ist und über den Umfang versetzte Schalen (3) aufweist, deren Konturen die Kommissuren der nativen Klappe umhüllen, wobei die Schalen in Kombination mit einer filtrierenden Membran (4) montiert sind, wobei die Letztere einerseits eine untere Schicht (4a), welche aus einem Maschennetz mit einer Porosität besteht, welche dafür angepasst ist, um Geweberückstände zu blockieren, während der Durchtritt des Blutstroms ermöglicht wird, und andererseits eine obere Schicht (4b) aufweist, welche aus einem weichen und ausdehnbaren polymeren Material ausgeführt ist, um als eine temporäre Klappe durch einfache Verformung zu wirken;
wobei die Vorrichtung **dadurch gekennzeichnet ist, dass** die Schichten (4a, 4b) durch Schweißung an der Basis der konischen Form und an seinem Umfang aneinander befestigt sind, wobei die Schweißung weich und schwammartig ist, um einen optimalen Sitz des Klappenfilters auf der Basis der Aortenwurzel bereitzustellen und um es dem Ende der konischen Form zu ermöglichen, geeignet zu sein, um schrittweise bis zum Einzug in das Innere des Katheters über das polymere Material geschlossen zu werden, indem es geschoben wird, um die eingefangenen Rückstände zu klistieren, wenn der Katheter (1) nach unten zwischen das polymere Material der Membran (4) und den polymeren Filter geschoben wird, bis das Ende der konischen Form erreicht ist.

2. Vorrichtung nach Anspruch 1, **dadurch gekennzeichnet, dass** die untere Maschenschicht an dem Ende des Körpers und in Verlängerung des Letzteren befestigt ist, während die obere Schicht, welche als Klappe dient, auf der Oberseite der konischen Form frei ist und offen bleibt.

3. Vorrichtung nach Anspruch 1 oder 2, **dadurch gekennzeichnet, dass** der Katheter (1) ein für Röntgenstrahlung undurchlässiges Ende aufweist, welches geeignet ist, das umliegende Gewebe während des Einführens und des Navigierens der Aorta zu schützen.

4. Vorrichtung nach Anspruch 3, **dadurch gekennzeichnet, dass** das Ende in das Innere des rohrförmigen Körpers der Anordnung (E), welche als Emboliefilter dient, zurückziehbar ist.

5. Vorrichtung nach Anspruch 4, **dadurch gekennzeichnet, dass** das Ende ein kleiner, aufblasbarer Ballon (6) ist, welcher mit steriler, für Röntgenstrahlung undurchlässiger Lösung gefüllt ist.

## Claims

1. Device for transcatheter insertion into the aortic root at the sinotubular junction by means of a guide wire and a catheter (1) with means for protecting the surrounding tissues, said device comprising an assembly (E) serving as a valvular embolic filter, mounted with the ability to slide in a guided manner inside said catheter (1), said assembly (E) having arrangements capable of forming, in the aortic root (AR), a safety chamber ensuring a valve function and a protective function against embolic accidents, said arrangements comprising a tubular body (2) secured, at one of its ends, to a part (2a) which is capable, as and when required, either of being deployed outside the catheter (1) or retracted inside the catheter (1), said part (2a) having filtration arrangements combined with means capable of reproducing the temporary valve function corresponding to opening during the systolic phase and to closing during the diastolic phase, in order to prevent any regurgitation of blood in a deployed position of said part for covering the native aortic valve with a seat secured in the aortic root at the sinuses of Valsalva without obstructing the blood flow; in which device said part (2a) is in the general shape of a cone and has angularly offset shells (3), of which the contours envelop the commissures of the native valve, said shells being mounted in combination with a filtering membrane (4), the latter comprising, on the one hand, a lower layer (4a) composed of a mesh network with a porosity suitable for blocking tissue debris while allowing the passage of the blood flow and, on the other hand, an upper layer (4b) made of a soft and extensible polymer material for acting as a temporary valve, by simple deformation; which device is **characterized in that** said layers (4a, 4b) are fixed together by welding at the base of the conical shape and on its perimeter, said weld being soft and spongy in order to provide an optimal seat for the valve filter on the base of the aortic root and in order to allow the end of said conical shape to be able to be closed progressively, until retraction inside the catheter, over the polymer material, by sliding it in order to clear the debris captured when the catheter (1) is slid downward between the polymer material of the membrane (4) and the polymer filter until reaching said end of said conical shape.

2. Device according to Claim 1, **characterized in that** the meshed lower layer is fixed to the end of the body and in a continuation of the latter, while the upper layer serving as a valve is free and remains open at the top of the conical shape.

3. Device according to Claim 1 or 2, **characterized in that** the catheter (1) has a radiopaque end capable of protecting the surrounding tissues during the introduction and navigation through the aorta.

4. Device according to Claim 3, **characterized in that** the end is retractable inside the tubular body of the assembly (E) serving as embolic filter.

5. Device according to Claim 4, **characterized in that** the end is an inflatable balloon (6) filled with sterile radiopaque solution.
